# EUROPÄISCHE PATENTSCHRIFT

(11) **EP 1 470 791 B1**
(45) Veröffentlichungstag und Bekanntmachung des Hinweises auf die Patenterteilung: **28.02.2007**
(21) Anmeldenummer: 03008840.5
(22) Anmeldetag: 25.04.2003
(51) Int. Cl.: A61B 19/00

(54) **Visualisierungsvorrichtung für kombinierte Patienten- und Objektbilddaten mit einer Eingabevorrichtung und Visualisierungsverfahren**
Visualisation apparatus with input means, for the combination of scanned and video images, and visualisation method
Appareil de visualisation de données d'imagerie médicale et d'images vidéo combinées, équipé de moyens de saisie d'entrées, et procédé de visualisation

(43) Veröffentlichungstag der Anmeldung: 27.10.2004
(73) Patentinhaber: BrainLAB AG, 85551 Kirchheim/Heimstetten (DE)
(72) Erfinder: Birkenbach, Rainer, 85445 Aufkirchen (DE); Schmidt, Robert, 81371 München (DE); Wohlgemuth, Richard, 81671 München (DE); Rossner, Holger-Claus, 85622 Feldkirchen (DE); Frielinghaus, Nils, 85551 Heimstetten (DE); Schaffrath, Claus, Dr., 81377 München (DE); Wörlein, Swen, 80617 München (DE)
(74) Vertreter: Schwabe - Sandmair - Marx

(56) Entgegenhaltungen:
- EP-A- 0 672 389
- EP-A- 1 327 421
- WO-A-96/20421
- WO-A-03/002011
- US-A- 5 694 142
- US-A- 5 765 561
- US-A- 6 038 467

## Beschreibung

Die Erfindung betrifft die Visualisierung für kombinierte Patienten- und Objektbilddaten in Systemen, wo Patientenbilddaten oder Objektbilddaten auf Videobilder projiziert werden.

Grundsätzlich weisen Vorrichtungen dieser Gattung eine Bildanzeigeeinrichtung, mindestens eine Videokamera und ein computergestütztes Navigationssystem auf, das die Raumpositionen der Anzeigevorrichtung und/oder der Videokamera sowie die Raumpositionen eines Patientenkörperteils über dort angebrachte Trackingeinrichtungen erfassen kann. Die Vorrichtungen dienen dazu, einen Mediziner bei der Diagnose bzw. bei der Behandlung eines Patienten visuell zu unterstützen. Es soll die Möglichkeit geschaffen werden, Bilder aus dem Inneren des Patienten mit herkömmlichen Videobildern zu verknüpfen, um Behandlung und Diagnose zu erleichtern.

EP-A-1 327 421 ist Teil des Stands der Technik gemäß Art. 54(3) EPÜ.

Die Bildanzeigevorrichtung und die Videokamera sind als tragbare Einheit ausgebildet.

Eingabe von Behandlungsplänen ist nicht offenbart.

Ein gattungsgemäßes, videobasiertes chirurgisches Zielsystem ist aus der US-A-5,765,561 bekannt, wobei unter anderem die Verwendung einer getrackten Videokamera vorgeschlagen wird, deren Bilder auf dem stationär angeordneten Bildschirm eines Navigationssystems mit zugeordneten Bildern aus Durchleuchtungs- bzw. Schichtbilderfassungsverfahren überlagert werden. Auch die US 5,715,836 beschreibt die Überlagerung von Bildern aus verschiedenen Quellen in einer Operations- bzw. Behandlungsumgebung. Die Bilder können aus der Computertomographie, Kernspintomographie, aus Ultraschall- oder Röntgenbildgeräten stammen. Auch hier werden die Bilder aber auf einem stationären Bildschirm ausgegeben.

WO 03/002 011 A1 beschreibt ein ähnliches System.

Eine Positionsverifizierung für Positionsmarkierungen in einem Videokamerabild, bei der Videokameras und Infrarotkameras kombiniert und deren Bilder überlagert werden, ist aus der DE 19 848 765 A1 bekannt. Auch hier werden die Bilder auf einem stationären Bildschirm ausgegeben.

Nachteilig an den oben beschriebenen Ausführungsformen ist insbesondere, dass der Arzt, der die Bilder betrachten möchte, immer wieder vom Patienten weg und zum Bildschirm des Navigationssystems hinblicken muss. Dabei kann er nicht mehr genau darauf achten, in welcher Stellung nun die Kamera steht, also von welcher Außenposition genau er die überlagerten Bilder erhält. Das System ist deshalb umständlich und ermöglicht keinen direkten Einblick in den Patientenkörperteil. Ferner müssen alle Eingaben, die der Arzt in das System zu tätigen gedenkt, an der Eingabestation des Navigationssystems bzw. des Behandlungsplanungssystems gemacht werden. Dabei muss sich der Arzt wiederum vom Patienten abwenden, und er kann die Wirkung der Eingaben nicht unmittelbar überprüfen.

Es ist die Aufgabe der vorliegenden Erfindung, eine Vorrichtung bzw. ein Verfahren zur visuellen Kombination von Patientenbilddaten aus Durchleuchtungs- bzw. Schichtbilderfassungsverfahren bzw. Objektbilddaten mit Videobildern bereitzustellen, welche die oben genannten Nachteile des Standes der Technik überwinden. Insbesondere soll es ermöglicht werden, direkt und unmittelbar in Ansicht des Patienten gewünschte Bildinformationen zu erhalten und dabei auch Einfluss auf die Behandlungsunterstützung auszuüben.

Diese Aufgabe wird dadurch gelöst, dass eine erfindungsgemäße Vorrichtung so ausgestaltet ist, dass die Bildanzeigevorrichtung und die Videokamera einander zugeordnet und als tragbare Einheit ausgebildet sind, und an der Bildanzeigevorrichtung eine Eingabevorrichtung vorgesehen ist, welche Eingaben für eine bildunterstützte Behandlung oder Behandlungsplanung ermöglicht.

Vorteilhafterweise ergibt sich dabei die Möglichkeit für den behandelnden Arzt, die Bildanzeigevorrichtung selbst mit zugeordneter Kamera vor das Patientenkörperteil zu halten und damit in unmittelbarem Aufblick auf den Patienten die gewünschten anatomischen Informationen auszulesen. Mit der erfindungsgemäßen Vorrichtung wird alleine durch die Zuordnung und Ausbildung von Videokamera und Bildanzeigevorrichtung als tragbare Einheit, ein weiterer Schritt in die Richtung getan, einen "gläsernen Patienten" zu schaffen, wobei die Bildanzeigevorrichtung lediglich vor das in Frage kommende Patientenkörperteil gehalten werden muss. Da die Kamerazuordnung zur Bildanzeigevorrichtung über die Trackingeinrichtung verfolgt wird, ist die Position der betrachteten Elemente immer bekannt, und das Navigationssystem hat Informationen darüber, wie und aus welcher Richtung der Patientenkörperteil angesehen wird. Insbesondere bei der Durchführung minimal invasiver Eingriffe, bei denen keine Freilegung des Eingriffsgebietes erfolgt, bietet die erfindungsgemäße Vorrichtung somit die Möglichkeit für den behandelnden Arzt, sich während der Operation nochmals genau über die Lage der Anatomieteile des Patienten bzw. anderer Objekte im Verhältnis zur Patientenanatomie in Kenntnis zu setzen. Auf der Bildanzeigevorrichtung können dabei natürlich auch getrackte, bzw. navigierte Instrumente angezeigt werden. Der Arzt muss seinen Blick nicht mehr von dem Patienten abwenden, um in erfindungsgemäßer Weise in das Innere des Patientenkörpers hineinzusehen.

Hinzu kommt noch der Vorteil, dass der Arzt durch die Erfindung dazu in die Lage versetzt wird, in direkter und unmittelbarer Ansicht des Patienten und mit Hilfe der Informationen aus der Bildanzeige über die Eingabevorrichtung noch Änderungen und Modifikationen vorzunehmen, die seine Arbeit unterstützen. Er kann dann die Wirkung seiner Modifikationen unmittelbar und in Ansicht des Patienten überprüfen, ohne sich vorher einer Eingabestation zuwenden zu müssen, was die Verifizierung oder Korrektur bestimmter Maßnahmen sehr vereinfacht und unmittelbarer gestaltet.

Bei einer bevorzugten Ausführungsform der Erfindung weist die Eingabevorrichtung elektromechanische Schalter auf. Diese Schalter können von jeder bekannten Art sein; vorstellbar sind Druckschalter, Richtungsdruckschalter, aber auch joystickartige Schalter, integriert oder kombiniert mit Druckschaltern. Es können mehrere Eingabevorrichtungen der vorher genannten Arten vorhanden sein. Möglich ist es ebenfalls, die Eingabevorrichtung zusätzlich oder alleine als Touchscreen auszubilden. Natürlich sind alle möglichen Eingabevorrichtungen realisierbar, auch fortgeschrittene wie Spracheingaben usw..

Gemäß einer bevorzugten Ausgestaltung der erfindungsgemäßen Vorrichtung ist dieser ein computergestütztes Behandlungsplanungs- und -unterstützungssystem zugeordnet, das Eingaben der Eingabevorrichtung verarbeitet. Die tragbare Einheit aus Bildanzeigevorrichtung und Videokamera wird dadurch zu einem Teil des Behandlungsplanungssystems sowie der Behandlungsunterstützung und erspart entsprechende separate Eingaben an separat dafür vorgesehenen Systemvorrichtungen.
Die erfindungsgemäße Vorrichtung kann eine kabelgestützte oder kabellose Datenübertragung zwischen Bildanzeigevorrichtung und dem computergestützten Behandlungsplanungsund Unterstützungssystem aufweisen, mittels der vorzugsweise auf eine Eingabe hin Objektinformationen übertragen werden. Solche Objektinformationen können sämtliche Arten von Bildinformationen umfassen, und die Übertragung kann in beiden Richtungen zum Informationsaustausch bzw. Abgleich erfolgen.

Die Erfindung betrifft ferner ein Verfahren zur visuellen Kombination von Patientenbilddaten aus Durchleuchtungs- bzw. Schichtbilderfassungsverfahren bzw. Objektbilddaten mit Videobildem auf einer Bildanzeigeeinrichtung, der mindestens eine Videokamera zugeordnet ist, bei dem ein computergestütztes Navigationssystem der Bildanzeigevorrichtung und/oder der Videokamera sowie die Raumpositionen eines Patientenkörperteils über dort angebrachte Trackingeinrichtungen erfasst, wobei die Bildanzeigevorrichtung und die Videokamera als tragbare Einheit ausgebildet sind, und bei dem Eingaben für eine bildunterstützte Behandlung oder Behandlungsplanung mittels einer Eingabevorrichtung an der Bildanzeigevorrichtung eingegeben werden. Die Vorteile des erfindungsgemäßen Verfahrens umfassen diejenigen, die oben im Hinblick auf die erfindungsgemäße Vorrichtung beschrieben wurden.

Bei einer Ausführungsform des Verfahrens gemäß der Erfindung wird ein computergestütztes Behandlungsplanungs- und -unterstützungssystem die Eingaben der Eingabevorrichtung verarbeiten. Dabei können behandlungsunterstützende Objekt- bzw. Bildinformationen über eine kabelgestützte oder kabellose Datenübertragung zwischen Bildanzeigevorrichtung und dem computergestützten Behandlungsplanungs- und -unterstützungssystem übertragen werden. Im Einzelnen können die Objekt- bzw. Bildinformationen eine oder mehrere der folgenden Informationen umfassen:
- Informationen über patientenspezifische, aus zwei- oder dreidimensionalen Bilddaten gewonnene Teile des Patientenkörpers,
- Informationen über nicht patientenspezifische, modellbasierte Objektdaten, insbesondere generische oder angepasste generische Körperteile, wie Knochen oder Weichteile,
- Informationen über Implantate,
- Messinformationen,
- Informationen über Medikamente, und speziell deren Verteilung, auch simulierte Verteilung,
- Informationen über Radiochirurgie- bzw. Strahlentherapiepläne,
- Informationen über Pläne zur bildgestützten Chirurgie,
- Informationen über Registrierungsmarker,
- Informationen über Instrumente,
- künstlich aus Bilddateninformationen erzeugte Röntgenbilder (DRRs = Digitally Reconstructet Radiographs) bzw. Informationen aus tatsächlichen Röntgenbildern.

Ein erfindungsgemäßes Verfahren kann so ausgestaltet sein, dass behandlungsunterstützende Bild- bzw. Objektdaten oder Behandlungspläne durch Eingaben an der Eingabevorrichtung geändert oder angepasst werden. Ferner kann die Bilddarstellung an der Bildanzeigeeinrichtung durch solche Eingaben geändert oder angepasst werden.

Gemäß einer weiteren Ausführungsform werden durch Planung vorhergesagte Änderungen am Patientenkörper durch Eingaben an der Eingabevorrichtung auf der Bildanzeigevorrichtung angezeigt. Dies ermöglicht eine unmittelbare Kontrolle der Planung in unmittelbarer Ansicht des Patientenkörperteils.

Eine weitere Ausführungsform des erfindungsgemäßen Verfahrens zeichnet sich dadurch aus, dass durch Eingaben an der Eingabevorrichtung Bilder auf der Bildanzeigevorrichtung gespeichert, kombiniert, zu Bildsequenzen oder Filmen zusammengefügt, versendet, abgespielt, voneinander subtrahiert oder mit Kommentaren versehen werden, wobei diese Tätigkeiten einzeln oder in jedweder Kombination durchgeführt werden können.

In besonders vorteilhafter Ausführung werden durch Eingabe an der Eingabevorrichtung andere Geräte in der Behandlungsumgebung gesteuert.

Es ist möglich, die Integration der Bildanzeigeeinrichtung durch ihre direkte Anbringung an ein Bilderfassungsgerät für das computergestützte Behandlungsplanungs- und -unterstützungssystem durchzuführen.

Allgemeine Ausführungsvarianten für die erfindungsgemäße Vorrichtung werden im Folgenden erläutert. Die Videokamera kann auf der Rückseite der Bildanzeigevorrichtung angeordnet sein. Dabei entsteht ein kompaktes Gerät; die Kamera kann vollständig aus dem Blickbereich des Benutzers herausgenommen werden. Grundsätzlich ist anzumerken, dass die Kamera an jeder Stelle der Rückseite der Bildanzeigevorrichtung angebracht oder in dieser integriert sein kann, z.B. auch im Randbereich der Bildanzeigevorrichtung. Die Videokamera steht bevorzugt in vorbestimmter bzw. systembekannter räumlicher Zuordnung zur Bildanzeigevorrichtung, und die Trackingeinrichtung ist an der Bildanzeigevorrichtung angeordnet. Durch die vorbestimmte bzw. systembekannte räumliche Zuordnung von Videokamera und Bildanzeigevorrichtung wird die Möglichkeit eröffnet, nur eines dieser beiden Geräte mittels der Trackingeinrichtung zu verfolgen, da man immer genau weiß, wo sich dann das andere Gerät befindet. Es steht dem Konstrukteur dann frei, die Trackingeinrichtung entweder an der Bildanzeigevorrichtung selbst oder an der Videokamera anzuordnen, je nachdem wie diese Trackingeinnchtung am Besten vom Navigationssystem erfasst werden kann. Bei optisch basierten Navigationssystemen kann die Trackingeinrichtung eine reflektierende oder aktiv abstrahlende Markeranordnung sein, während es natürlich im Rahmen der vorliegenden Erfindung auch möglich ist, die Navigation über ein Magnettrackingsystem durchzuführen, bei dem Spulen in einem erzeugten Magnetfeld verfolgt werden. Was die Navigations- bzw. Trackingsysteme betrifft, die mit reflektierenden Markeranordnungen arbeiten, so sind solche einsetzbar, die mit unsichtbarem Licht (z. B. Infrarot) und einer entsprechenden Lichtquelle arbeiten, aber auch solche, die eine Videotechnologie verwenden, die mit sichtbarem Licht (Raumlicht) arbeitet. Letztere Systeme verwenden optische Marker, die sichtbares Licht reflektieren, wobei die Analyse der Markerkennung jedoch ausschließlich im Videobild vorgenommen wird.

Die Bildanzeigevorrichtung ist leicht und gut handhabbar auszugestalten, indem sie als tragbarer Flachbildschirm, insbesondere LCD-Flachbildschirm konstruiert wird. Dieser Schirm kann natürlich, wie vorher bemerkt, die Touchscreen-Funktion aufweisen. Die Energieversorgung der erfindungsgemäßen Vorrichtung kann durch eigene Energieversorgung (Batterie bzw. Akku) oder über Kabel erfolgen. Falls eine drahtlose, insbesondere Funkübertragung für die Daten verwendet wird, eignet sich besonders eine eigene Energieversorgung für die Vorrichtung, damit die weitgehendste Handhabungsfreiheit erreicht wird.

Die Videokamera kann eine kleine Blende und eine geringe Schärfentiefe aufweisen, damit nur ein kleiner Bereich des erfassten Bildes in der Fokalebene liegt. Mit einer solchen Kamera ist es möglich, den Abstand zwischen dem Bild und der Fokalebene zu ermitteln. Wenn die Raumposition der Kamera aus dem Navigationssystem her bekannt ist und der Abstand zur Bildebene ebenfalls bekannt ist, kann das dem Navigationssystem angeschlossene Computersystem die räumliche Position des Videobildes in Echtzeit errechnen. Damit wird eine Zuordnung von Videobild und Bilddaten aus einer vorher durchgeführten Durchleuchtungs- bzw. Schichtbilderfassung optimal möglich, ebenso wie die Zuordnung von Objektdaten.

Die zu überlagernden einzelnen Bilddaten bzw. Objektdaten werden vorteilhafterweise in größerer Position auf der Bildanzeigevorrichtung so angepasst, dass eine korrekte bzw. gewünschte Größenzuordnung entsteht, also zum Beispiel eine 1:1 Darstellung der Bilder in Überdeckung. Im Navigationssystem bzw. im Behandlungsplanungs- und -unterstützungssystem, welches als ein Teil des Navigationssystems oder separat und diesem zugeordnet zur Verfügung gestellt werden kann, kann eine Konturen-Matching-Einheit bereitgestellt werden, mittels der die Überdeckung der dargestellten Bilddaten realisiert wird, insbesondere über ein Außenkonturen-Matching für den Patientenkörperteil.

An der Kamera bzw. in deren Umgebung an der Bildanzeigevorrichtung ist vorteilhafterweise eine Beleuchtungsvorrichtung für den Patientenkörperteil vorgesehen, speziell beispielsweise als Ringlicht (LEDs, Lampen, Leuchtstoffröhren).

Die Erfindung wird im Weiteren anhand einer Ausführungsform näher erläutert. In den hierzu bereitgestellten Zeichnungen zeigen:
- Figur 1: eine schematische Darstellung für die Verwendung einer erfindungsgemäßen Vorrichtung zur Ansicht eines Patienten-Kniegelenks in der Aufsicht;
- Figur 2: eine seitliche Ansicht der Vorrichtung nach Figur 1 mit schematisch dargestelltem Navigationssystem;
- Figur 3: ein Ablaufdiagramm für den Betrieb der erfindungsgemäßen Vorrichtung, und
- Figur 4 und 5: perspektivische Vorder- und Rückansicht einer erfindungsgemäßen Vorrichtung.

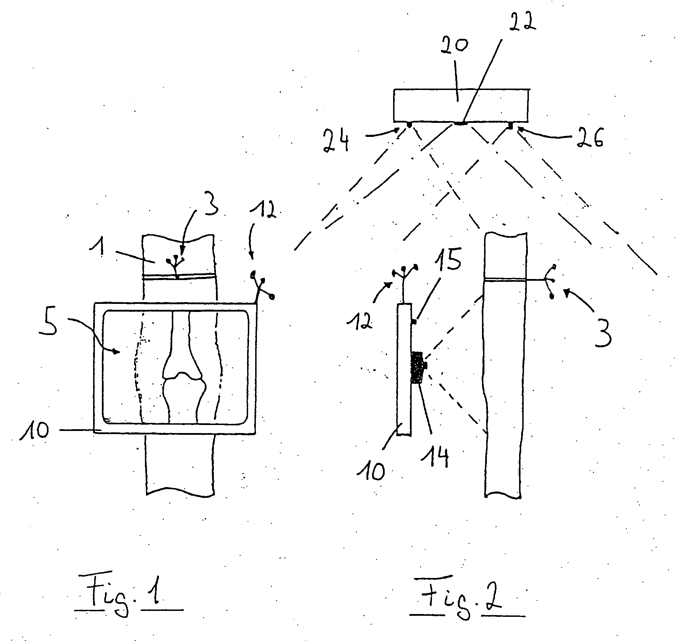

In der Figur 1 ist schematisch eine erfindungsgemäße Vorrichtung dargestellt, mit der beispielsweise in das Innere eines Gelenks eines Patienten Einsicht genommen werden kann. Das Bein 1 des Patienten soll am Gelenk 5 untersucht werden. Hierzu wird der erfindungsgemäß ausgestaltete Flachbildschirm 10 direkt vor das Gelenk gehalten, und auf dem Schirm 10 erscheint dann ein Bild, das sowohl Bildinformationen aus dem Videokamerabild der Kamera 14 (Figur 2) enthält als auch Bildinformationen über die innere anatomische Struktur, welche beispielsweise per Funkübertragung von einem Navigationsgerät übermittelt werden. Dazu ist vorab von dem Patienten bzw. von dessen Bein 1 eine Schichtbildaufnahme (MR- oder CT-Aufnahme) erstellt worden.

Wie besser in Figur 2 deutlich wird, befindet sich die Kamera 14 fest angebracht an der Rückseite des Bildschirms 10.

Möglich wird diese Art der Bilddarstellung dadurch, dass die Position der Kamera 14 bzw. des Bildschirms 10 im Raum um das Bein 1 des Patienten herum mittels einer Trackingeinrichtung ermittelt wird. Diese Trackingeinrichtung ist im vorliegenden Fall eine optische Trackingeinrichtung, nämlich eine Markeranordnung 12, deren Position mittels des schematisch dargestellten Navigationssystems 20 erfasst wird. Das Navigationssystem 20 umfasst hierzu beispielsweise zwei beabstandete Infrarot-Kameras 24, 26 und einen Infrarot-Lichtabstrahler 22. Aus den beiden Bildern der Infrarot-Kamera 24, 26 kann die dreidimensionale Raumposition der Markeranordnung 12 und damit auch des Bildschirms 10 und der fest installierten Kamera 14 ermittelt werden. Es ist ferner möglich die Raumposition des Beines 1 durch eine entsprechende Markeranordnung 3 im Raum zu ermitteln.

Wenn nun die Position von Bildschirm 10 und Kamera 14 bekannt ist, kann in folgender Weise die Position der aktuellen Bildebene erfasst werden. Die Kamera 14 hat nämlich eine kleine Blende und eine geringe Schärfentiefe, so dass nur das Bild in einer bestimmten Fokalebene scharf erscheint. Es lässt sich hierdurch die Position dieser Bildebene ermitteln und auf dem Bildschirm 10 ein Bild aus dem CT- oder MR-Aufnahmeverfahren übertragen, welche ebenfalls gerade in dieser Bildebene liegt. Wichtig ist hierbei, dass das Abbildungsverhältnis des Videobildes in einem 1: 1-Maßstab zum Schichtaufnahmebild angezeigt wird. Wenn nun mit der erfindungsgemäßen Vorrichtung das Gelenk eines Patienten betrachtet wird, sieht man beispielsweise am oberen und unteren Bildschirmrand die Konturen des Patientenbeines. Mit dieser Information kann nunmehr das Bild aus dem Schichtaufnahmeverfahren so angepasst werden, dass es gerade über dem Videobild liegt, um einen realistischen Eindruck zu gewährleisten. Der Betrachter des Bildschirms 10 kann dann auf dem Bildschirm sowohl das Videobild, also ein echtes Bild, ansehen, als auch das "innere" Bild aus dem Schichtaufnahmeverfahren, und zwar in jeweils gewünschter Tiefe und in jeweils eingestellter Transparenz für beide Bilder. Diese Einstellungen können über Steuerungsorgane, und andere erfindungsgemäße Eingaben (die weiter unten genauer erörtert werden) getroffen werden, wie eines davon mit dem Bezugszeichen 15 angedeutet ist.

Die erfindungsgemäße Vorrichtung gibt damit dem Betrachter erstmals die Möglichkeit, in direktem Aufblick auf den Patienten in Echtzeit in den inneren Behandlungsbereich hineinzusehen und die Behandlung entsprechend zu planen oder anzupassen. Er muss seinen Blick nicht mehr vom Patienten abwenden und auf einen fest installierten Navigationsbildschirm sehen, was insbesondere von Vorteil ist, wenn die Reaktionen des Patienten auf bestimmte ärztliche Maßnahmen überprüft werden sollen. Da die "inneren" Bilder aus den Durchleuchtungs- bzw. Schichtbildaufnahmeverfahren so gestaltet werden können, dass sie virtuell 3-D-Ansichten der inneren Körperteile darstellen, kann das Bild insgesamt sehr anschaulich gemacht werden.

Obwohl also nicht vor Ort ein Durchleuchtungs- bzw. Schichtbild aufgenommen wird, entsteht somit ein virtuell "gläserner Patient".

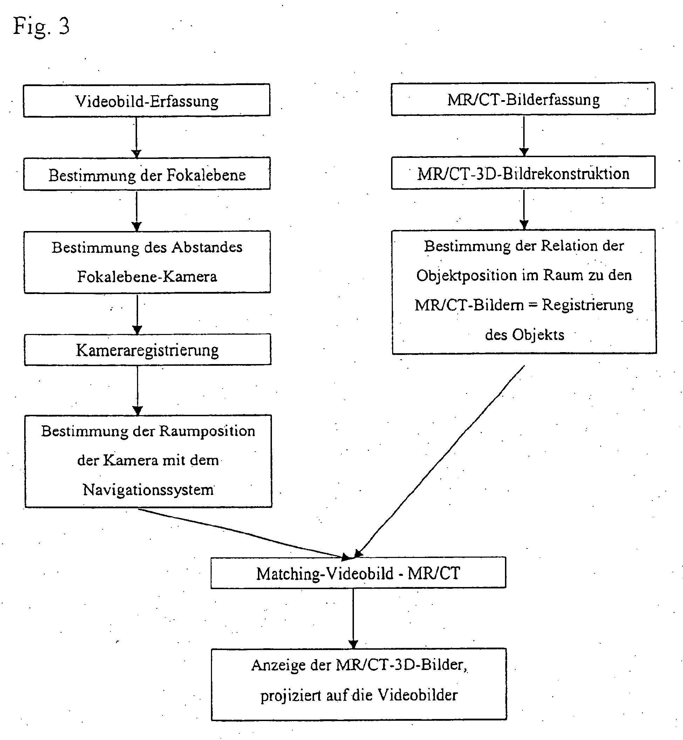

In der Figur 3 ist, bis zur Bildüberlagerung und für diesen speziellen Fall, der Ablauf eines Verfahrens aufgezeigt, wie es mit der erfindungsgemäßen Vorrichtung durchgeführt wird, insbesondere wird hierbei die vom Computer des Navigationssystems durchgeführte Tätigkeit beleuchtet. Die obere linke Seite des Ablaufdiagramms betrifft die Vorgänge bei der Videobilderfassung, während auf der rechten Seite die Bilderfassung für das Körperinnere erläutert wird, und zwar am Beispiel einer MR-/CT-Bilderfassung. Bei der Videobilderfassung wird zunächst die Fokalebene bestimmt, wie dies vorher schon erläutert wurde. Danach erfolgt eine Bestimmung des Abstandes der Fokalebene zur Kamera, worauf die Kamera mittels ihrer Trackingeinrichtung im Navigationssystem registriert wird. Es ist nun die Bestimmung der Raumposition der Kamera gegenüber dem Navigationssystem bekannt.

Was die MR-/CT-Bilderfassung betrifft, so wird diese zunächst vorab erfolgen, worauf aus den erhaltenen Daten eine dreidimensionale Bildrekonstruktion erfolgt. Dann wird die Registrierung des Objektes, beispielsweise eines Patientenkörperteils, im Raum durchgeführt, d. h. es erfolgt eine Bestimmung der Relation der Objektposition im Raum zu den MR-/CT-Bildern. Realisiert werden kann dies durch die räumliche Erfassung einer am Objekt befindlichen Markierungsanordnung.

Wenn die oben aufgeführten Schritte der Videobilderfassung und MR-/CT-Bildverarbeitung durchgeführt worden sind, erfolgt nunmehr ein Videobild-/MR-/CT-Bild-Matching, d. h. die beiden Bilder werden computergesteuert in Übereinstimmung gebracht, was die Bildposition und die Bildgröße betrifft. Danach können die MR-/CT-3D-Bilder projiziert auf die Videobilder auf der Bildanzeigevorrichtung überlagert werden und den behandelnden Arzt bei der Diagnose bzw. Behandlung unterstützen.

Insbesondere können die übertragenden Objekt- bzw. Bildinformationen künstlich aus Bilddateninformationen erzeugte Röntgenbilder (DRRs = Digitally Reconstructed Radiographs) sein, oder Informationen aus tatsächlichen Röntgenbildern. In diesem Fall existiert beispielsweise von einem Patienten ein diagnostischer Scan, vorzugsweise ein CT-Scan, und der tragbare Flachbildschirm dient sozusagen als künstlicher Röntgenfilm, dass heißt das Tracking- bzw. Navigationssystem ermittelt die Position des Bildschirms und kennt die Position des Patienten und kann nun virtuelle durch den Patienten hindurch "Strahlen senden", die wie bei einem Röntgengerät dann zu einem röntgenähnlichen Bild führen. Dadurch erhält der Benutzer die Möglichkeit, virtuell eine Röntgenquelle in der Hand zu halten und aus sämtlichen Betrachtungswinkeln Röntgenbilder zu erhalten.

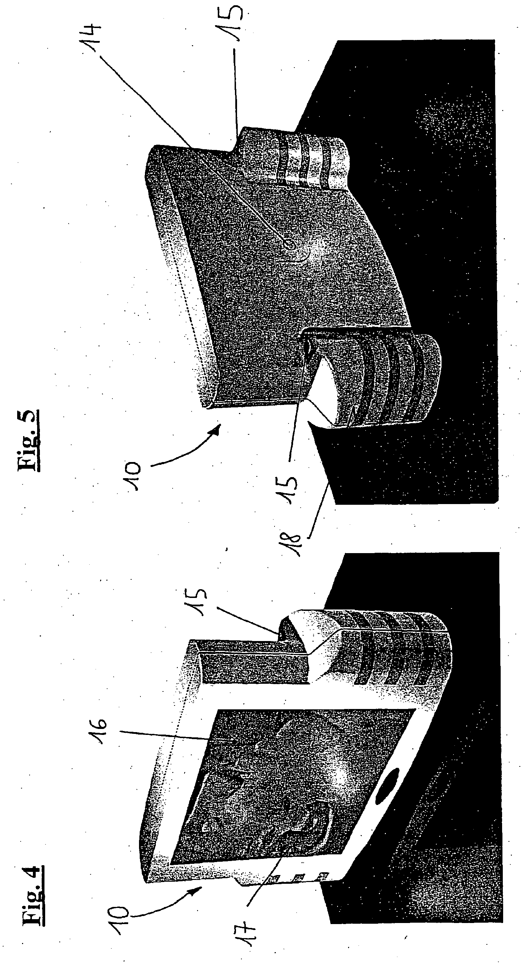

Die Figuren 4 und 5 zeigen in perspektivischer Einstellung eine Ausführungsform der erfindungsgemäßen Vorrichtung von der Vorder- und der Rückseite her. Die Bildanzeigevorrichtung 10 weist in diesem Fall einen Bildschirm 16 auf, der als Touchscreen ausgebildet sein kann. Auf dem Bildschirm 16 ist das gerade aufgenommene Videobild eines Patienten zu sehen, und beispielsweise im Ohr sind patientenspezifische Objekte, nämlich innere Strukturen 17 eingeblendet. Der tragbare Bildschirm 10 weist zusätzlich noch Eingabeknöpfe 15 auf, die oben am Handgriff 18 bzw. an den beidseitigen Handgriffen angeordnet sind. Die Kamera 14 ist hinten mittig in das Gerät eingelassen.

Mit diesen Emgabeknöpfen 15 und/oder mit dem Touchscreen 16 können nun Eingaben getätigt werden, welche die erfindungsgemäße Vorrichtung zu einem multifunktionellen Gerät machen, das bei der Behandlungsplanung bzw. Behandlungsunterstützung eingesetzt werden kann. Die Vorrichtung 10 ist z.B. per Funk mit einem hier nicht dargestellten, üblichen Behandlungsunterstützungs- bzw. Behandlungsplanungssystem verbunden, um Daten auszutauschen. Dabei können im Einzelnen folgende Tätigkeiten einzeln oder in Kombination durchgeführt werden:

Dargestellte Objekte können ein- bzw. ausgeschaltet werden, d.h. sie werden sichtbar oder unsichtbar gemacht. Parameter von dargestellten Objekten können verändert werden, beispielsweise die Farbe, die Transparenz etc.. Es kann ein- bzw. ausgezoomt werden, und die Helligkeit bzw. der Kontrast können am Bildschirm geregelt werden. Objekte können markiert bzw. angewählt und verschoben werden, beispielsweise zur Planung oder zur Modifikation eines Behandlungsplanes. Landmarken oder Messstrecken können gesetzt werden. Es ist möglich, Screenshots der Bilddarstellung abzuspeichern oder zur Dokumentation Bilder zu versenden. Filmaufnahmen aus mehreren erfassten Bildern können abgespielt, gestartet oder angehalten werden.

Der Arbeitsabstand kann verändert werden, d.h. die "virtuelle Schärfentiefe", wobei festgelegt wird, in welchem Abstand Objekte sichtbar sind. Planungsmöglichkeiten bestehen, beispielsweise die für die Planung von Trajektorien und Ebenen (Knochenschnitt). Es ist möglich, bestimmte interessierende Bereiche an der Bilddarstellung separat hervorzuheben, d.h. eine Art Spotlight auf bestimmte Gebiete zu richten.

Ferner können die Eingabemittel 15, 16 zur Steuerung anderer Geräte, z.B. Navigationssystem, Behandlungsroboter, Mikroskop, C-Bogen, Ultraschall, Raumlicht etc. verwendet werden. Das Bild kann eingefroren werden, insbesondere während die Instrumente weiter navigiert und aufprojiziert werden. Eine Differenzbildung von zwei oder mehreren Bildern, vorzugsweise als Vorher/Nachher-Bild kann auf eine Eingabe hin dargestellt werden; ebenfalls kann eine Undo/Redo Funktion bereitstehen.

Panoramabilder können erzeugt werden, beispielsweise um ein komplettes, längeres Patientenkörperteil abzubilden (Bein).

Es ist möglich, Texteingaben vorzunehmen, z.B. zum Bezeichnen von Punkten und zur Eingabe von Kommentaren zu bestimmten dargestellten Merkmalen. Durch Eingabesteuerung kann die Bildanzeigevorrichtung als intelligente Digitalkamera, auch Videokamera zu Dokumentationszwecken verwendet werden und es sind Erweiterungssätze denkbar, die beispielsweise Hilfen aus Datenspeichern oder dem Internet bereitstellen.

Ein weiteres Anwendungsgebiet erschließt sich, wenn durch die Eingaben Vorschauen von Körpermodifikationen eingeblendet werden können, um dem Arzt direkt vor Ort und in Ansicht des Patienten zu zeigen, wie das Ergebnis seiner Behandlungsplanung aussehen könnte. Unter dem Begriff Objekte ist im Rahmen der Erfindung ganz allgemein eine breite Vielfalt von Bilddateninformationen zu verstehen, und zwar über tatsächliche, körperliche Gegenstände, aber auch über virtuelles Bildinformationsmaterial. Solche Objektinformationen können aus patientenspezifischen, zweidimensionalen oder dreidimensionalen Daten gewonnen werden, die beispielsweise aus Bilderfassungsverfahren stammen, wie CT, MR, funktioneller MR, Ultraschall, PET, SPECT, Röntgenaufnahmen etc.. Es ist aber ebenso möglich, modellbasierte Daten zu verwenden, also ohne Daten des aktuellen Patienten zu arbeiten. Hierfür kommen beispielsweise Knochen in Frage (Röhrenknochen, Schädel, Knochenstücke MKG etc.), aber auch Weichteile (Hirn, Bänder, Flüssigkeiten etc.). Diese Daten können dann in der Größe angepasst werden bzw. werden aus einem Satz vorhandener Daten bzw. vorhandener Objekte die passenden ausgewählt.

Sonstige Objekte bzw. Objektinformationen betreffen Implantate (Hüfte, Knie, Zähne, Marknägel, Schrauben, Platten) insbesondere Implantate im Wirbelsäulenbereich (Schrauben, künstliche Bandscheiben, Cages, interne/externe Fixateure, oder auch im Gebiet des Hirns (Oberflächenelektroden, Deep Brain Stimulator, Clips, Coils, Katheter etc.). Die Implantate können ebenfalls aus vorbestimmten Größen ausgewählt und virtuell an der Bildanzeigevorrichtung eingesetzt werden, um zu sehen, welches Implantat wo am Besten passt und wie das Ergebnis aussieht.
Es ist ferner möglich, andere Objekte bzw. Objektinformationen einzublenden, wie z.B. Messinformationen (Lineale, Abstände zum Zielgebiet, etc.). Zeitsimulationen über die Injektion verabreichter Medikamente bzw. deren Verteilung über die Zeit können per Eingabe angefordert und visuell dargestellt werden, ebenso wie Radiochirurgie- bzw. Strahlentherapiepläne und deren vorhersagbare Auswirkungen. Ferner können die Objekte natürlich Registrierungsmarker und Instrumente umfassen (Endoskop, Säge, Bohrer, Ahle, etc.).

Insgesamt lässt sich sagen, dass alle interessierenden Informationen über Objekte bzw. Informationen, die Objekte betreffen, zwischen der Bildanzeigeeinrichtung und Unterstützungssystemen ausgetauscht werden können, wobei der Datentausch natürlich auch Modifikationen an solchen Objekten und Objektinformationen beinhaltet.

Es ist beispielsweise ferner möglich, Implantate (Haut, Knochen) in einer ersten Region zu erkennen und zu gewinnen, und dann in einer zweiten Region einzufügen. Eventuell können die gewonnen Implantate dabei auch navigiert werden. Im Bereich der Wirbelsäulenchirurgie können Befestigungselemente wie Befestigungsstangen, die üblicherweise per Hand gebogen und angepasst werden, unter Kontrolle durch die Bildanzeigevorrichtung und mit den Informationen über den Patienten virtuell angepasst werden, d.h. geplant werden. Dabei wird dann in Ansicht der Patientenanatomie schon die richtige Form des Befestigungselementes bestimmt, das dann so gefertigt werden kann. Ein zeitraubendes Anpassen bei geöffneten Patienten ist nicht mehr notwendig.

Es kann ferner die Form eines Instruments aufgenommen, registriert und dann navigiert werden und es besteht die Möglichkeit, mit Modellen zu trainieren bzw. Simulationen durchzuführen.

Wenn ein Touchscreen verwendet wird, können auf diesem Touchscreen Einzeichnungen bzw. Markierungen vorgenommen werden, was beispielsweise in der Craniotomie und für Elektroden vorteilhaft sein kann.

Ein weiteres Anwendungsfeld betrifft die Anpassung der Bilderfassungsdaten, die beispielsweise vorab von dem Patienten durch CT, MR oder ähnliche Verfahren erfasst wurden. Die Videokamera der Bildanzeigevorrichtung kann beispielsweise Texturen des Patienten als Videobild aufnehmen und damit die Daten aus der Bilderfassung verbessern bzw. "verschönern" . Beispielsweise zeigen CT- oder MR-Schichtbildaufnahmen keine besonders guten Patienten-Oberflächenstrukturen. Diese Oberflächenstrukturen sind aber aus dem Videobild bekannt und können die Schichtbilddaten ergänzen. In der plastischen Chirurgie kann dies bei der Planung von Eingriffen und insbesondere bei der visuellen Vorab-Simulation von Ergebnissen dieser Eingriffe von großem Vorteil sein.

Die Helligkeit des Videobildes kann zur Anpassung der Objektdarstellung verwendet werden, und in einer weiteren Ausführungsform besteht die Möglichkeit, die Bildanzeigevorrichtung direkt an dem Bildaufnahmegerät für die inneren Strukturen des Patienten (CT, MR, fMRI, etc.) zu befestigen, damit eine Zuordnung bzw. Registrierung der unterschiedlichen Bilddaten vereinfacht wird.

Schließlich ist es noch möglich, die Bedienung der Eingabevorrichtungen dadurch zu vereinfachen, dass die tragbare Einheit aus Bildanzeigevorrichtung und Videokamera zeitweise auf einem dafür vorgesehenen Arm mit Halterung abstellbar bzw. befestigbar ist, wobei dieser Arm auch beweglich sein kann.

## Patentansprüche

1. Verfahren zur visuellen Kombination von Patientenbilddaten aus Durchleuchtungs- bzw. Schichtbilderfassungsverfahren bzw. Objektbilddaten mit Videobildern auf einer Bildanzeigevorrichtung (10), der mindestens eine Videokamera (14) zugeordnet ist, wobei das Verfahren folgende Schritte umfasst:
- die Verwendung einer Bildanzeigevorrichtung (10) und einer Videokamera (14), die als tragbare Einheit ausgebildet sind;
- die Erfassung der Raumpositionen der Bildanzeigevorrichtung (10) und/oder der Videokamera (14) von einem computergestützten Navigationssystem;
- die Erfassung der Raumpositionen eines Patientenkörperteils (1) über dort angebrachte Trackingeinrichtungen (12, 3) von diesem computergestützten Navigationssystem;
- die Eingabe von Eingaben für eine bildunterstützte Behandlung oder Behandlungsplanung mittels einer Eingabevorrichtung (15) an der Bildanzeigevorrichtung (10);
wobei
- Behandlungspläne durch die Eingaben an der Eingabevorrichtung (15) geändert oder angepasst werden, und dass
- die Eingaben der Eingabevorrichtung (15) von einem computergestützten Behandlungsplanungs- und -unterstützungssystem verarbeitet werden.

2. Verfahren nach Anspruch 1, bei dem behandlungsunterstützende Objekt- bzw. Bildinformationen über eine kabelgestützte oder kabellose Datenübertagung zwischen Bildanzeigevorrichtung (10) und dem computergestützten Behandlungsplanungs- und - unterstützungssystem übertragen werden.

3. Verfahren nach Anspruch 1, bei dem die übertragenen Objekt- bzw. Bildinformationen eine oder mehrere der folgenden Informationen umfassen:
- Informationen über patientenspezifische, aus zwei- oder dreidimensionalen Bilddaten gewonnene Teile des Patientenkörpers,
- Informationen über nicht patientenspezifische, modellbasierte Objektdaten, insbesondere generische oder angepasste generische Körperteile, wie Knochen oder Weichteile,
- Informationen über Implantate,
- Messinformationen,
- Informationen über Medikamente, und speziell deren Verteilung, auch simulierte Verteilung,
- Informationen über Radiochirurgie- bzw. Strahlentherapiepläne,
- Informationen über Pläne zur bildgestützten Chirurgie,
- Informationen über Registrierungsmarker,
- Informationen über Instrumente,
- künstlich aus Bilddateninformationen erzeugte Röntgenbilder (DRRs = Digitally Reconstructet Radiographs) bzw. Informationen aus tatsächlichen Röntgenbildern.

4. Verfahren nach einem der Ansprüche 2 oder 3, bei dem behandlungsunterstützende Bild- bzw. Objektdaten oder Behandlungspläne durch Eingaben an der Eingabevorrichtung (15) geändert oder angepasst werden.

5. Verfahren nach einem der Ansprüche 1 bis 4, bei dem die Bilddarstellung an der Bildanzeigeeinrichtung durch Eingaben an der Eingabevorrichtung (15) geändert oder angepasst werden.

6. Verfahren nach einem der Ansprüche 1 bis 5, bei dem durch Planung vorhergesagte Änderungen am Patientenkörper durch Eingaben an der Eingabevorrichtung (15) auf der Bildanzeigevorrichtung angezeigt werden.

7. Verfahren nach einem der Ansprüche 1 bis 6, bei dem durch Eingaben an der Eingabevorrichtung (15) Bilder auf der Bildanzeigevorrichtung (10) gespeichert, kombiniert, zu Bildsequenzen oder Filmen zusammengefügt, versendet, abgespielt, voneinander subtrahiert oder mit Kommentaren versehen werden, wobei diese Tätigkeiten einzeln oder in jedweder Kombination durchgeführt werden können.

8. Verfahren nach einem der Ansprüche 1 bis 7, bei dem durch Eingaben an der Eingabevorrichtung (15) andere Geräte in der Behandlungsumgebung gesteuert werden.

9. Verfahren nach einem der Ansprüche 1 bis 8, **dadurch gekennzeichnet, dass** die Integration der Bildanzeigeeinrichtung (10) durch ihre direkte Anbringung an ein Bilderfassungsgerät für das computergestützte Behandlungsplanungs- und - unterstützungssystem erfolgt.

## Claims

1. A method for visually combining patient image data from transillumination and/or tomographic imaging methods and/or object image data with video images on an image display device (10) with which at least one video camera (14) is associated, wherein the method comprises the steps of:
- using an image display device (10) and a video camera (14) which are formed as a portable unit;
- detecting the spatial positions of the image display device (10) and/or the video camera (14), using a computer-assisted navigation system;
- detecting the spatial positions of a part (1) of the patient's body via tracking means (12, 3) attached to it, using said computer-assisted navigation system;
- inputting inputs for image-assisted treatment or treatment planning by means of an input device (15) on the image display device (10);
wherein
- treatment plans are changed or adapted by the inputs at the input device (15), and
- the inputs of the input device (15) are processed by a computer-assisted treatment planning and assisting system.

2. The method according to claim 1, wherein treatment-assisting object and/or image information is transmitted via cable-assisted or cable-free data transmission between the image display device (10) and the computer-assisted treatment planning and assisting system.

3. The method according to claim 1, wherein the transmitted object and/or image information includes one or more of the following types of information:
- information on patient-specific parts of the patient's body obtained from two-dimensional or three-dimensional image data;
- information on non-patient-specific, model-based object data, in particular generic or adapted generic parts of the body such as bones or soft tissues;
- information on implants;
- measurement information;
- information on medicines, and specifically their distribution, also simulated distribution;
- information on radiosurgical and radiotherapeutic plans;
- information on plans for image-assisted surgery;
- information on registering markers;
- information on instruments;
- x-ray images artificially generated from image data information (DDRs = digitally reconstructed radiographs) and/or information from actual x-ray images.

4. The method according to any one of claims 2 or 3, wherein treatment-assisting image or object data or treatment plans are changed or adapted by inputs at the input device (15).

5. The method according to any one of claims 1 to 4, wherein image representation at the image display means is changed or adapted by inputs at the input device (15).

6. The method according to any one of claims 1 to 5, wherein changes to the patient body predicted by planning are displayed on the image display device by inputs at the input device (15).

7. The method according to any one of claims 1 to 6, wherein images on the image display device (10) are stored, combined, spliced into image sequences or films, forwarded, played back, subtracted from each other or annotated, by inputs at the input device (15), and wherein these actions can be performed individually or in any combination.

8. The method according to any one of claims 1 to 7, wherein other apparatus in the treatment environment are controlled by inputs at the input device (15).

9. The method according to any one of claims 1 to 8, wherein the image display means (10) is integrated by directly attaching it to an imaging apparatus for the computer-assisted treatment planning and assisting system.

## Revendications

1. Procédé pour la combinaison visuelle sur un dispositif d'affichage d'images (10) auquel est associée au moins une caméra vidéo (14) de données d'images de patients provenant de procédés de radiographie et/ou de procédés d'acquisition d'images tomographiques ou de données d'images d'objets avec des images vidéo, le procédé comprenant les étapes suivantes :
- l'utilisation d'un dispositif d'affichage d'images (10) et d'une caméra vidéo (14) qui sont réalisés sous forme d'unité portable,
- l'acquisition des positions spatiales du dispositif d'affichage d'images (10) et/ou de la caméra vidéo (14) par un système de navigation assisté par ordinateur,
- l'acquisition par ce système de navigation assisté par ordinateur des positions spatiales d'une partie du corps du patient (1) par l'intermédiaire de dispositifs de poursuite (12, 3) qui y sont implantés et
- l'introduction au moyen d'un dispositif d'entrée (15) se trouvant sur le dispositif d'affichage d'images (10) d'indications pour un traitement ou pour une planification de traitement assisté par des images,
des plans de traitement étant modifiés ou adaptés par les indications introduites dans le dispositif d'entrée (15) et
les indications introduites dans le dispositif d'entrée (15) étant traitées par un système assisté par ordinateur de planification du traitement et d'assistance au traitement.

2. Procédé selon la revendication 1, dans lequel des informations d'objets ou d'images d'assistance au traitement sont transmises par l'intermédiaire d'une transmission de données filaire ou sans fil entre le dispositif d'affichage d'images (10) et le système assisté par ordinateur de planification du traitement et d'assistance au traitement.

3. Procédé selon la revendication 1, dans lequel les informations d'objets ou d'images transmises comprennent une ou plusieurs des informations suivantes :
- informations concernant des parties du corps du patient qui sont spécifiques au patient et qui sont obtenues à partir de données d'images bidimensionnelles ou tridimensionnelles,
- informations concernant des données d'objets non spécifiques au patient et basées sur des modèles, en particulier des parties du corps qui sont génériques ou génériques adaptées, telles que des os ou des parties molles,
- informations concernant des implants,
- informations de mesure,
- informations concernant des médicaments, et en particulier leur répartition et aussi leur répartition simulée,
- informations concernant des plans de radiochirurgie ou des plans de thérapie par rayons,
- informations concernant des plans de chirurgie assistée par des images,
- informations concernant des repères d'enregistrement,
- informations concernant des instruments
- images radiographiques générée artificiellement à partir d'informations de données d'images (DRR = Digitally Reconstructed Radiographs) et/ou informations provenant d'images radiographiques réelles.

4. Procédé selon l'une quelconque des revendications 2 ou 3, dans lequel des données d'images ou d'objets d'assistance au traitement ou des plans de traitement sont modifiés ou adaptés par des indications introduites dans le dispositif d'entrée (15).

5. Procédé selon l'une quelconque des revendications 1 à 4, dans lequel la représentation des images sur le dispositif d'affichage d'images est modifiée ou adaptée par des indications introduites dans le dispositif d'entrée (15).

6. Procédé selon l'une quelconque des revendications 1 à 5, dans lequel des modifications sur le corps du patient prédites par une planification sont affichées sur le dispositif d'affichage d'images par des indications introduites dans le dispositif d'entrée (15).

7. Procédé selon l'une quelconque des revendications 1 à 6, dans lequel des images sont mémorisées dans le dispositif d'affichage d'images (10) par des indications introduites dans le dispositif d'entrée (15), sont combinées, sont regroupées en séquences d'images ou en films, sont expédiées, sont visionnées, sont soustraites l'une de l'autre ou sont munies de commentaires, ces activités pouvant avoir lieu individuellement ou dans n'importe quelle combinaison.

8. Procédé selon l'une quelconque des revendications 1 à 7, dans lequel d'autres équipements se trouvant dans l'environnement de traitement sont commandés par des indications introduites dans le dispositif d'entrée (15).

9. Procédé selon l'une quelconque des revendications 1 à 8, **caractérisé en ce que** l'intégration du dispositif d'affichage d'images (10) a lieu par sa mise en place directe sur un appareil d'acquisition d'images du système assisté par ordinateur de planification du traitement et d'assistance au traitement.
